(19) Europäisches Patentamt

European Patent Office

Office européen des brevets

(11) **EP 4 691 973 A1**

(12) **EUROPEAN PATENT APPLICATION**
published in accordance with Art. 153(4) EPC

(43) Date of publication:
**11.02.2026 Bulletin 2026/07**

(21) Application number: **24779036.3**

(22) Date of filing: **29.02.2024**

(51) International Patent Classification (IPC):
***C01B 21/064*** (2006.01)   ***A61K 8/19*** (2006.01)
***A61Q 1/12*** (2006.01)

(52) Cooperative Patent Classification (CPC):
**A61K 8/19; A61Q 1/12; C01B 21/064**

(86) International application number:
**PCT/JP2024/007518**

(87) International publication number:
**WO 2024/202897 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.03.2023 JP 2023049896**

(71) Applicant: **TOKUYAMA CORPORATION**
**Yamaguchi 745-8648 (JP)**

(72) Inventors:
• **KAKIGI, Tomoyuki**
**Shunan-shi, Yamaguchi 745-8648 (JP)**
• **DAIKI, Shota**
**Shunan-shi, Yamaguchi 745-8648 (JP)**
• **ABURATANI, Makoto**
**Shunan-shi, Yamaguchi 745-8648 (JP)**

(74) Representative: **Steffens, Adrian**
**Boehmert & Boehmert**
**Anwaltspartnerschaft mbB**
**Pettenkoferstraße 22**
**80336 München (DE)**

(54) **HEXAGONAL BORON NITRIDE POWDER AND PRODUCTION METHOD THEREFOR**

(57)    Provided is hexagonal boron nitride powder that makes it possible to easily provide a cosmetic providing both airy, light texture and moist feel sensed in spreading thereof. Hexagonal boron nitride powder having a tap bulk density of not more than 0.08 g/cm$^3$ and a shearing cohesion of not less than 3.5 kPa can attain the object, the shearing cohesion being calculated from a powder yield locus obtained through a powder bed shear stress analyzing test. The hexagonal boron nitride powder can be easily produced by a method for producing hexagonal boron nitride powder including a heating step of filling, with raw material powder containing coarse hexagonal boron nitride powder, a heating container so that a bulk density thereof becomes 0.16 g/cm$^3$ to 0.23 g/cm$^3$ and heating the raw material powder at a maximum temperature of 1700°C to 2100°C for not less than 3 hours under a nitrogen atmosphere, the coarse hexagonal boron nitride powder having a specific surface area of 12 m$^2$/g to 30 m$^2$/g and an eluted boron content of 1% to 15%. (Fig. 1)

FIG. 1

3.00kV x500 SE                                    100um

## Description

Technical field

The present invention relates to hexagonal boron nitride powder suitable for an application as a cosmetic and a method for producing the same.

Background art

Hexagonal boron nitride powder, which is a white pigment having a layered structure with a hexagonal system, is excellent in lubricating properties, spreadability, coverage performance, and chemical stability, and thus is used as raw materials for a variety of cosmetics. Therefore, it has been reported that an attempt has been made to further improve demanded properties of hexagonal boron nitride powder as raw materials for cosmetics. For example, Patent Literature 1 discloses that setting an aspect ratio, an average particle diameter, a specific surface area, a graphitization index, and a tap density (tap bulk density) of hexagonal boron nitride powder to certain ranges improves applying smoothness and concealing performance.

Among a variety of demanded properties, the "moist feel" has been attracting attention in recent years. Non-Patent Literature 1 discloses that a difference between a static friction force and a dynamic friction force contributes to the moist feel. Patent Literature 2 discloses hexagonal boron nitride powder providing improved moist feel while maintaining appropriate lubricating properties by setting an average particle diameter to 2.5 $\mu$m to 7.0 $\mu$m and setting a content of the aggregated particles having particle diameters of not less than 36 $\mu$m to not more than 10.0% by volume.

Citation List

Patent Literature

Patent Literature 1
Japanese Patent Application Publication, Tokukai, No. 2018-108970
Patent Literature 2
International Publication No. WO 2019/172440

Non-patent Literature

Non-patent Literature 1
Bulletin of the Chemical Society of Japan, 2020, 93, 399-405

Summary of invention

Technical Problem

As described above, also the conventional hexagonal boron nitride powder is found to exert a certain effect of enhancing the moist feel, which is a demanded property as raw materials for cosmetics. However, there has been a problem in achieving both high moist feel and other demanded properties. For example, hexagonal boron nitride powder providing high moist feel has a high adhesion property to the skin and exhibits a high packing property as powder, whereas hexagonal boron nitride powder providing airy feel, as disclosed in paragraph [0026] of Patent Literature 1, contains a lot of air and has a low tap bulk density, resulting in a low packing property, so that it has been difficult to achieve both moist feel and airy feel of cosmetics with use of the hexagonal boron nitride powder. The hexagonal boron nitride powder disclosed in Patent Literature 2, which has a limitation on an aggregated particle content to not more than 10.0% by volume, has an improved packing property of particles and is excellent in the moist feel, but is poor in the airy feel. Thus, in cosmetics requiring airy, light feel, such as loose foundations, it has been necessary to adjust compositions of the components other than the hexagonal boron nitride powder in the cosmetics in order to achieve both the airy, light texture and the moist feel sensed in spreading thereof.

Thus, the present invention has been made in light of the aforementioned background, and it is an object thereof to provide hexagonal boron nitride powder that makes it possible to easily provide a cosmetic providing both the airy, light texture and the moist feel sensed in spreading thereof.

Solution to problem

As a result of a diligent study, the present inventors have found that a cosmetic containing hexagonal boron nitride powder adjusted to have specific values of a tap bulk density and a shearing cohesion calculated from a powder yield locus obtained through a powder bed shear stress analyzing test provides moist feel and airy feel, both of which are favorable, and the present inventors have completed the present invention.

That is, the present invention is hexagonal boron nitride powder having a tap bulk density of not more than 0.08 $g/cm^3$ and a shearing cohesion of not less than 3.5 kPa, the shearing cohesion being calculated from a powder yield locus obtained through a powder bed shear stress analyzing test. In the hexagonal boron nitride powder, in a powder residue on a sieve having a mesh size of 25 $\mu$m, a proportion of structures made of aggregates of primary hexagonal boron nitride particles having an elongation of not less than 0.80 is preferably not less than 13%. Further, the hexagonal boron nitride powder preferably has a particle diameter D50 of 15 $\mu$m to 30 $\mu$m and an average aspect ratio of 15 to 20, and preferably has a particle diameter D90 of 37 $\mu$m to 52 $\mu$m.

The hexagonal boron nitride powder is preferably used for a cosmetic application. An aspect of the present invention is a cosmetic containing the hexagonal boron nitride powder, in particular, a loose foundation containing the hexagonal boron nitride powder.

Furthermore, an aspect of the present invention is a producing method including filling a heating container with raw material powder containing coarse hexagonal boron nitride powder having a specific surface area of 12 $m^2$/g to 30 $m^2$/g and an eluted boron content of 1% to 15% so that a bulk density thereof becomes 0.16 $g/cm^3$ to 0.23 $g/cm^3$ and heating the raw material powder at 1700°C to 2100°C for not less than 3 hours under a nitrogen atmosphere.

Advantageous effects of invention

Hexagonal boron nitride powder in accordance with the present invention facilitates provision of a cosmetic excellent in both the airy, light texture sensed in use thereof and the moist feel sensed in spreading thereof. Thus, for example, in cosmetics having a low oil content and providing high airy feel, such as loose foundations, conventionally, the moist feel has been poor and the dryness has been likely to feel, whereas use of hexagonal boron nitride powder in accordance with the present invention facilitates preventing dryness while maintaining airy feel, and thus makes it possible to increase a degree of freedom in the design of cosmetics.

Brief description of drawings

Fig. 1 is a view illustrating one example of a structure made of aggregates of primary hexagonal boron nitride particles having an elongation of not less than 0.80 contained in hexagonal boron nitride powder in accordance with Example 1 of the present invention.

Fig. 2 is a view illustrating a long-axis diameter a and a short-axis diameter b of the structure made of the aggregates of the primary hexagonal boron nitride particles in accordance with the present invention.

Description of embodiments

Hexagonal boron nitride powder

Hexagonal boron nitride powder in accordance with the present invention has a feature of having a tap bulk density of not more than 0.08 $g/cm^3$ and a shearing cohesion of not less than 3.5 kPa, the shearing cohesion being calculated from a powder yield locus obtained through a powder bed shear stress analyzing test.

The tap bulk density refers to a mass per unit volume of hexagonal boron nitride powder and is an index indicating how tight of a state of the particles is. In other words, the tap bulk density can be also regarded as an index indicating a volume of the voids contained in the powder, and a lower value thereof indicates that the powder contains voids in a higher volume. When the powder contains the voids in a higher volume, the powder provides higher airy texture. Setting the tap bulk density to not more than 0.08 $g/cm^3$ facilitates provision of a cosmetic having the airy, light feel. The tap bulk density is further preferably not more than 0.06 $g/cm^3$. Meanwhile, the tap bulk density is preferably not less than 0.01 $g/cm3$ from a viewpoint of handling. Setting the tap bulk density to not less than 0.01 $g/cm^3$ facilitates processing and mixing into, for example, cosmetics.

The shearing cohesion refers to a shearing stress which is calculated from a powder yield locus indicating a relationship between a vertical stress and a shearing stress that occurs when shearing is carried out while applying the vertical stress, with use of hexagonal boron nitride powder as a powder bed and in which no vertical stress acts. That is, it can be regarded as an index that indicates a cohesion between particles in the hexagonal boron nitride powder and that is not affected by a force of application of the hexagonal boron nitride powder and a surface state of an application target. When the powder is applied to the skin, a higher shearing cohesion of the powder makes it easier to impart the moist feel to a cosmetic. The reason for this is not clear, but the present inventors consider as follows. That is, when the powder is applied to the skin, a user potentially senses, in addition to a cohesion between the skin and the powder, a cohesion between the particles, and this is considered to be important for providing the moist feel. It is expected that conventional hexagonal boron nitride powder providing high moist feel contains a slight amount of water and has a high wettability to the skin and an improved adhesion property, resulting in high moist feel sensed by the user. **In** such conventional hexagonal boron nitride powder providing high moist feel, although the appearance thereof is not significantly changed, the water connects the particles, and thus fluidity of the particles is slightly reduced, resulting in a high shearing cohesion. However, it has been impossible to increase the volume of the voids, since the particles are connected by the slight amount of water. As a result, the tap bulk density becomes higher, resulting in failure to provide the airy feel. In contrast, hexagonal boron nitride powder in accordance with the present invention provides airy feel in the application to the skin due to a low tap bulk density. Further, when a user spreads the hexagonal boron nitride powder after application thereof to the skin, the hexagonal boron nitride powder provides a feel similar to that of the above powder containing a slight amount of water, since the fluidity of the particles is slightly reduced due to a high cohesion between the particles. As a result, it is presumed that the user perceives the hexagonal boron nitride powder in accordance with the present invention as having a high wettability, and senses high moist feel.

The hexagonal boron nitride powder in accordance with an aspect of the present invention has the shearing cohesion of not less than 3.5 kPa, preferably 3.7 kPa, further preferably not less than 4.0 kPa. Setting the shearing cohesion to not less than 3.5 kPa can provide moist texture in the application to the skin. The upper limit of the shearing cohesion is not particularly set, but it may be typically not more than 6.0 kPa, in particular, not more than 5.0 kPa.

It is preferable that in hexagonal boron nitride powder in accordance with an aspect of the present invention, in a powder residue of the hexagonal boron nitride powder which is left on a sieve having a mesh size of 25 $\mu$m, a proportion of structures made of aggregates of primary hexagonal boron nitride particles having elongations of not less than 0.80 is not less than 13%. When the contained structures made of aggregates of primary hexagonal boron nitride particles having elongations of not less than 0.80 account for not less than 13% of the group of the relatively large particles that have not passed through the sieve having a mesh size of 25 $\mu$m, it is easier to provide the hexagonal boron nitride powder having a tap bulk density of not more than 0.08 g/cm$^3$ and a shearing cohesion of not less than 3.5 kPa, the shearing cohesion being calculated from a powder yield locus obtained through a powder bed shear stress analyzing test. The reason for this is not clear, but the present inventors consider as follows.

That is, when the group of the relatively large particles that have not passed through the sieve having a mesh size of 25 $\mu$m contains many structures having relatively high elongations, the structures penetrate between the aggregated particles and between the aggregated particles and the single particles and thus contain many voids. This facilitates adjustment of the tap bulk density to a low level. It is presumed that the high elongations of the structures cause the structures to engage with each other in spreading of the powder, thus generating an instantaneous large frictional force, and thus the shearing cohesion becomes higher, resulting in high moist feel. Further, it is presumed that the breakage of the structures in spreading of the powder causes a decrease in a frictional force, and this variation in the frictional force also contributes to a higher shearing cohesion.

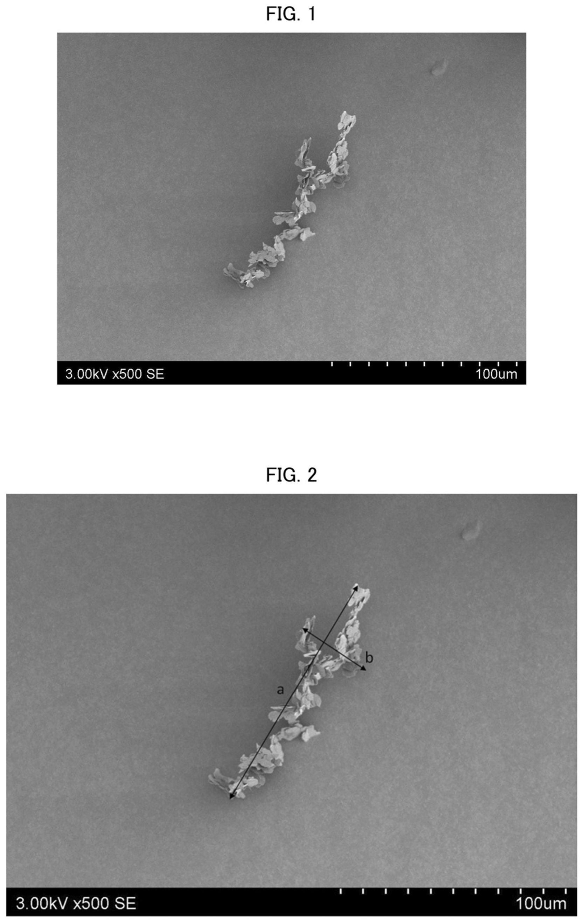

Note that the elongation of the structure in the present invention refers to an index indicating the slenderness of the structure, and a greater elongation indicates that the structure is more elongated. As illustrated in Fig. 2, on the assumption that the longest straight line of the straight lines that connect edges and edges of the structure is regarded as a long axis and the longest straight line of the straight lines that are orthogonal to the long axis and connect edges and edges of the structure is regarded as a short axis, the elongation of the structure is calculated by the following formula from the length of the long axis (long-axis diameter a) and the length of the short axis (short-axis diameter b). The long-axis diameter a and the short-axis diameter b can be measured from images taken with use of a scanning electron microscope.

$$\text{Elongation} = 1 - (b/a)$$

A proportion of the structures made of aggregates of primary hexagonal boron nitride particles having elongations of not less than 0.80 in the powder residue on the sieve having a mesh size of 25 $\mu$m is preferably not less than 13%, further preferably, not less than 16%. The upper limit of the proportion is not particularly set, and all of the powder residue on the sieve having a mesh size of 25 $\mu$m may be made up of the structures made of the aggregates of the primary hexagonal boron nitride particles having elongations of not less than 0.80. However, the proportion may be typically not more than 25%, in particular, not more than 20%.

Hexagonal boron nitride powder in accordance with an aspect of the present invention preferably has a particle diameter D50 of not less than 15.0 $\mu$m, further preferably, not less than 20.0 $\mu$m. Setting the particle diameter D50 to not less than 15.0 μm makes a cohesion between single particles moderate, and thus facilitates provision of high spreadability and airy feel in a cosmetic containing hexagonal boron nitride powder in accordance with an aspect of the present invention. The particle diameter D50 is preferably not more than 40.0 μm, further preferably not more than 35.0 μm, still further preferably not more than 30.0 μm, particularly preferably not more than 25.0 μm. Setting the particle diameter D50 to not more than 40.0 μm facilitates provision of smoothness and prevention of luster, in a cosmetic containing hexagonal boron nitride powder in accordance with an aspect of the present invention.

Hexagonal boron nitride powder in accordance with an aspect of the present invention preferably has a particle diameter D90 of 37.0 μm to 52.0 μm, further preferably 37.0 μm to 45.0 μm, still further preferably 38.0 μm to 42.0 μm. The particle diameter D90 falling within the above range facilitates increasing the moist feel and airy feel while preventing luster, in a cosmetic containing hexagonal boron nitride powder in accordance with an aspect of the present invention.

In an aspect of the present invention, the particle diameter D50 and the particle diameter D90 of the hexagonal boron nitride powder mean a cumulative 50% value (particle diameter D50) based on volume and a cumulative 90% value (D90) based on volume, respectively, in a particle diameter distribution curve.

In hexagonal boron nitride powder in accordance with an aspect of the present invention, a proportion of the powder residue on the sieve having a mesh size of 25 μm is preferably not less than 40% by mass, and further preferably, not less than 50% by mass. The upper limit of the proportion of the powder residue on the sieve having a mesh size of 25 μm is not particularly limited, but is typically not more than 70% by mass, in particular, not more than 60% by mass. The proportion of the powder residue on the sieve having a mesh size of 25 μm can be calculated by sieving all or some of the hexagonal boron nitride powder with use of a sieve having a mesh size of 25 μm and using a mass of the powder sieved and a mass of the powder residue on the sieve.

The primary particles of hexagonal boron nitride powder in accordance with an aspect of the present invention have an aspect ratio of preferably 15.0 to 20.0, further preferably, 17.0 to 18.0. The aspect ratio of each primary particle falling within the above range facilitates spreading with favorable spreadability while preventing luster in the application to the skin. When the aspect ratio of the primary particle is less than 15.0, there is a risk that a cosmetic containing hexagonal boron nitride powder in accordance with an aspect of the present invention provides resistance feel in the application thereof and the spreadability thereof is reduced. Meanwhile, when the aspect ratio of the primary particle exceeds 20.0, there is a risk that a cosmetic containing hexagonal boron nitride powder in accordance with an aspect of the present invention exhibits luster after the application thereof to the skin.

In hexagonal boron nitride powder in accordance with an aspect of the present invention, an eluted boron content is preferably not more than 20 ppm. Setting the eluted boron content to not more than 20 ppm makes it possible to reduce an impact on the skin in the application to the skin of the cosmetic containing hexagonal boron nitride powder in accordance with an aspect of the present invention.

Method for producing hexagonal boron nitride powder

Hexagonal boron nitride powder in accordance with an aspect of the present invention can be easily obtained by, although not particularly limited to, subjecting coarse hexagonal boron nitride powder obtained under specific conditions to a heating treatment under specific conditions. Specifically, one possible method for producing hexagonal boron nitride powder involves a heating step of filling, with raw material powder containing coarse hexagonal boron nitride powder, a heating container so that a bulk density thereof becomes 0.16 g/cm$^3$ to 0.23 g/cm$^3$ and heating the raw material powder at 1700°C to 2100°C for not less than 3 hours under a nitrogen atmosphere, the coarse hexagonal boron nitride powder having a specific surface area of 12 m$^2$/g to 30 m$^2$/g and an eluted boron content of 1% to 15%.

Method for producing coarse hexagonal boron nitride powder

As the coarse hexagonal boron nitride powder, conventional one can be used without any particular limitation, provide that it has a specific surface area of 12 m$^2$/g to 30 m$^2$/g and an eluted boron content of 1% to 15%. For example, the coarse hexagonal boron nitride powder can be produced by the melamine method in which a raw material mixture containing an oxygen-containing boron compound and a nitrogen-containing organic compound is heated under a nitrogen atmosphere.

Examples of the oxygen-containing boron oxide include diboron trioxide (boron oxide), diboron dioxide, tetraboron trioxide, metaboric acid, perboric acid, and orthoboric acid. Generally, boric acid and boron oxide, which are easily available are suitably used. The average particle diameter of the oxygen-containing boron compound is, although not particularly limited, preferably 30 μm to 800 μm, further preferably 100 μm to 500 μm, in terms of manipulability and reactivity. The average particle diameter of more than 30 μm makes it possible to reduce a moisture absorbency and facilitate handling. Setting the average particle diameter to not more than 800 μm makes it possible to prevent unevenness of the raw material components, and thus facilitating control of the reaction.

Examples of the nitrogen-containing organic compound include cyandiamide, ammeline, melamine, and urea.

Generally, melamine, which is easy to handle, is suitably used.

**In** the raw material mixture for producing the coarse hexagonal boron nitride powder, the element ratio (B/N) of boron/nitrogen is preferably 0.2 to 1.0, further preferably 0.25 to 0.75. The raw material mixture can be obtained by measuring out predetermined amounts of the above oxygen-containing boron compound and nitrogen-containing organic compound and mixing them. The mixing method is not particularly limited, provided that the components can be uniformly dispersed, and can be carried out with use of a general mixer such as a vibrating mill, a bead mill, a ball mill, and a mixer.

Nitride powder can be obtained by heating the raw material mixture described above under a nitrogen atmosphere so that the mixture reacts. The heating temperature is not particularly limited, provided that it is not less than 1600°C, but it is preferable that the temperature be increased so as to gradually reach respective temperature ranges of a reaction process and a firing process into which the temperature profile to the maximum temperature is divided, and the heating be maintained. In the reaction process, a range of 700°C to 1200°C is preferable, and a range of 800°C to 1100°C is more preferably. Setting the heating temperature in the reaction process to the range above makes it possible to efficiently proceed with the reaction while preventing the raw material mixture from being volatilized.

The maximum temperature in the firing process is preferably 1600°C to 2000°C, more preferably 1700°C to 1800°C. Setting the maximum temperature to not lower than 1600°C makes it likely that progression of a nitridation reaction of the oxygen-containing boron compound is efficiently completed for a short time. Further, setting the maximum temperature to not higher than 2000°C prevents yellowing and a decrease in transparency due to the nitrogen defect and prevents the specific surface area from being too small, thus making it likely that the particles efficiently grow in the heating treatment described later.

The retention time in the reaction process is preferably 1 hour to 5 hours, more preferably 2 hours to 4 hours. A too short retention time in the reaction process may leave raw materials unreacted, and a too long retention time in the reaction process leads to reduced productivity. The retention time in the firing process is preferably 1 hour to 6 hours, more preferably 2 hours to 5 hours. A too short retention time in the firing process may lead to insufficient growth of the particles, reduced crystallinity, and difficulty in controlling the particle diameter, and a too long retention time in the firing process leads to reduced productivity.

The nitride powder obtained by the nitridation reaction typically contains solidified bodies of not less than several hundreds of $\mu$m, and thus is preferably disintegrated with a force strong enough to crush the solidified bodies. The disintegration of the solidified bodies also makes it possible to make the specific surface area of the coarse hexagonal boron nitride powder moderate. Further, the disintegration of the solidified bodies makes it possible to efficiently carry out the washing step and the classification step described later. The disintegration method is not particularly limited, provided that the solidified bodies can be disintegrated, and the disintegration can be suitably carried out with use of, for example, a stone mill type pulverizer, a ball mill, a dry-type jet mill, a mortar, a roll crusher, and a wet-type rotary disc mill.

Washing using pure water or the like may be carried out in order to remove the impurities in disintegration powder obtained in the disintegration step and adjust an eluted boron content in the coarse hexagonal boron nitride powder. The washing method and an amount of the pure water or the like used for the washing are not particularly limited, and a known method can be used without limitation. The drying method for the disintegration powder after the washing is not particularly limited, and a known method can be used without limitation. Examples of a usable drying device include a shelf-type drier, a fluidized-bed drier, a spray drier, a rotarytype drier, and a belt-type dryer. **In** a case where an eluted boron content in the coarse hexagonal boron nitride powder becomes too low by, for example, excessive washing, the eluted boron content in the coarse hexagonal boron nitride powder may be adjusted by adding an oxygen-containing boron compound, such as boron oxide.

Classification may be carried out for removal of the solidified bodies remaining in the disintegration powder obtained in the disintegration step. The method for the classification is not particularly limited, and a known method can be applied without limitation. Specific examples of the method include a vibrating sieve machine, a wet-type sieve machine, an air classification machine, a cyclone separator, and a hydrocyclone separator. The mesh size of the sieve is preferably 25 $\mu$m to 90 $\mu$m, more preferably 25 $\mu$m to 45 $\mu$m. Setting the mesh size to not more than 90 $\mu$m makes it possible to increase a proportion of the single particles in the powder and efficiently generate the structures in the heating step described later. When the mesh size is less than 25 $\mu$m, the process takes a longer time, and thus it is preferably not less than 25 $\mu$m.

Method for producing hexagonal boron nitride powder

Hexagonal boron nitride powder in accordance with an aspect of the present invention can be easily produced by a method for filling a heating container with raw material powder containing the coarse hexagonal boron nitride powder having a specific surface area of 12 m²/g to 30 m²/g and an eluted boron content of 1% to 15% so that a bulk density thereof becomes 0.16 g/cm³ to 0.23 g/cm³ and heating the raw material powder at 1700°C to 2100°C for not less than 3 hours under a nitrogen atmosphere.

The reason why this provides hexagonal boron nitride powder in accordance with the present invention is unclear. However, the present inventors consider as follows. That is, heating the coarse hexagonal boron nitride powder having an eluted boron of 1% to 15%, under a nitrogen atmosphere causes the coarse hexagonal boron nitride powder to be heated in the presence of a slight amount of boric acid on the particle surfaces, and thus the nitridation reaction proceeds again on the particle surfaces, and portions of the primary particles are connected to each other while the particles are growing, resulting in generation of the structures. Setting the bulk density in the heating to 0.16 g/cm$^3$ to 0.23 g/cm$^3$ makes it possible to slightly orient the particles while leaving moderate gaps between the particles. It is presumed that although the connection occurs at the ends of surfaces a that are highly likely brought into contact by the orientation, the presence of the voids prevents the connection between the structures in a direction of the c axis, so that the structures having an elongation of not less than 0.80 are obtained, and consequently, the hexagonal boron nitride powder having a tap bulk density of not more than 0.08 g/cm$^3$ and a shearing cohesion of not less than 3.5 kPa is easily obtained, the shearing cohesion being calculated from a powder yield locus obtained through a powder bed shear stress analyzing test.

The coarse hexagonal boron nitride powder has a specific surface area of 12 m$^2$/g to 30 m$^2$/g, preferably 15 m$^2$/g to 28 m$^2$/g, further preferably 20 m$^2$/g to 25 m$^2$/g. The eluted boron content in the coarse hexagonal boron nitride powder is 1% to 15%, preferably 2% to 10%, further preferably 3% to 5%. When the specific surface area and the eluted boron content of the coarse hexagonal boron nitride powder fall within the above ranges, it is possible to achieve efficient particle growth and generation of structures by the heating treatment. That is, the specific surface area falling within the above range makes it possible to cause nitrogen to flow into the powder while leaving sufficient reaction points on the primary particles of the coarse hexagonal boron nitride powder. In addition, it is presumed that the eluted boron content that serves as a boron source falls within the above range, thus making it likely that the particle growth proceeds and facilitating formation of structures having great elongations. Setting the eluted boron content in the coarse hexagonal boron nitride powder to not more than 15% facilitates reducing the eluted boron content in the hexagonal boron nitride powder to not more than 20 ppm.

The raw material powder may contain the coarse hexagonal boron nitride powder, and the raw material powder may consist of the coarse hexagonal boron nitride powder alone or may contain other components to an extent that does not impair the effects of the present invention. However, the raw material powder preferably consists of the coarse hexagonal boron nitride powder alone, since it becomes easier to omit a washing operation after heating described later or to simplify a classification operation described later.

The heating temperature is preferably 1700°C to 2100°C, further preferably, 1750°C to 2000°C, still further preferably, 1800°C to 1950°C. Setting the temperature for heating the coarse hexagonal boron nitride powder to not lower than 1700°C facilitates forming the structures while efficiently causing the particles to grow, and increasing the shearing cohesion. Setting the heating temperature to not higher than 2100°C prevents yellowing caused by a nitrogen defect and facilitates provision of hexagonal boron nitride powder having an appropriate color tone as a cosmetic application. In addition, it is possible to adjust the particle diameter of the hexagonal boron nitride powder through the heating temperature, and the heating temperature falling within the above range facilitates adjustment of the particle diameter of the hexagonal boron nitride powder thus obtained to a range suitable for a cosmetic application.

The hexagonal boron nitride powder obtained by subjecting the coarse hexagonal boron nitride powder to the heating treatment by the method described above may be washed after the heating in order to remove the impurities. The washing can be carried out, for example, with use of pure water or the like, and the washing method and an amount of the pure water or the like used for the washing are not particularly limited, and a known method can be used without limitation.

Hexagonal boron nitride powder obtained by subjecting the coarse hexagonal boron nitride powder to the heating treatment by the method described above may be classified in order to, for example, remove the impurities and control the particle diameter D90 described above. The method for the classification is not particularly limited, and a known method can be applied without limitation. Specific examples of the method include a vibrating sieve machine, a wet-type sieve machine, an air classification machine, a cyclone separator, and a hydrocyclone separator. The mesh size of the sieve is preferably 40 μm to 90 μm, more preferably 45 μm to 63 μm. Setting the mesh size to not more than 90 μm makes it possible to reduce luster of the hexagonal boron nitride powder. When the mesh size is too small, the process takes a longer time.

Cosmetic

Hexagonal boron nitride powder in accordance with the present invention has applications that are not particularly limited, but is preferably used for cosmetic applications. In particular, the hexagonal boron nitride powder is favorably applied to solid or powder-like cosmetics. One possible aspect of the present invention is a cosmetic containing the hexagonal boron nitride powder, in particular, a solid or powder-like cosmetic containing the hexagonal boron nitride powder. Containing the hexagonal boron nitride powder in accordance with the present invention in a cosmetic makes it possible to easily achieve both moist feel and airy feel. Thus, both the moist feel and the airy feel conventionally cannot be achieved or are achieved by adjusting the compositions of the other components according to need, whereas the use of hexagonal boron nitride powder in accordance with the present invention makes it easier to achieve both the moist feel and the airy feel than conventional art, and thus makes it possible to increase the degree of freedom in the design of cosmetics. Examples of the cosmetics include loose foundations and face powders. A content proportion of the hexagonal boron nitride powder to the entire cosmetic is not particularly limited, but is preferably set to 0.1% by mass to 50% by mass. Containing, in a cosmetic, the present invention within the above range makes it easier to feel an effect of improving the moist feel and airy feel of the cosmetic.

Aspects of the present invention can also be expressed as follows:

As described above, hexagonal boron nitride powder in accordance with Aspect 1 of the present invention has a tap bulk density of not more than 0.08 g/cm$^3$ and a shearing cohesion of not less than 3.5 kPa, the shearing cohesion being calculated from a powder yield locus obtained through a powder bed shear stress analyzing test.

Hexagonal boron nitride powder in accordance with Aspect 2 of the present invention may be configured, in Aspect 1 above, such that in a powder residue on a sieve having a mesh size of 25 $\mu$m, a proportion of structures made of aggregates of primary hexagonal boron nitride particles having an elongation of not less than 0.80 is not less than 13%.

Hexagonal boron nitride powder in accordance with Aspect 3 of the present invention may be configured, in Aspect 1 or 2, to have a particle diameter D50 of 15 $\mu$m to 30 $\mu$m and an average aspect ratio of 15 to 20.

Hexagonal boron nitride powder in accordance with Aspect 4 of the present invention may be configured, in any one of Aspects 1 to 3, to have a particle diameter D90 of 37 $\mu$m to 52 $\mu$m.

Hexagonal boron nitride powder in accordance with Aspect 5 of the present invention may be configured, in any one of Aspects 1 to 4, to be used for a cosmetic application.

A cosmetic in accordance with Aspect 6 of the present invention contains the hexagonal boron nitride powder in accordance with any one of Aspects 1 to 5.

A loose foundation in accordance with Aspect 7 of the present invention contains the hexagonal boron nitride powder in accordance with any one of Aspects 1 to 5.

A method for producing hexagonal boron nitride powder in accordance with Aspect 8 of the present invention includes a heating step of filling, with raw material powder containing coarse hexagonal boron nitride powder, a heating container so that a bulk density of the raw material powder becomes 0.16 g/cm$^3$ to 0.23 g/cm$^3$ and heating the raw material powder at a maximum temperature of 1700°C to 2100°C for not less than 3 hours under a nitrogen atmosphere, the coarse hexagonal boron nitride powder having a specific surface area of 12 m$^2$/g to 30 m$^2$/g and an eluted boron content of 1% to 15%.

Examples

Examples are described in order to specifically describe the present invention, but the present invention is not limited to these Examples. The measurement of each item in Examples and Comparative Examples was carried out by the following method.

(1) Tap bulk density of hexagonal boron nitride powder

The tap bulk density (g/cm$^3$) was measured with use of Tap Denser KYT-5000 (available from SEISHIN ENTERPRISE CO., LTD.). Specifically, a 100-mL sample cell was filled with 100 mL of hexagonal boron nitride powder and was tapped under conditions of a tap speed of 120 times/minute, a tap height of 5 cm, and a number of times of the tap of 500 times. The weight thereof was then measured, and the tap bulk density thereof was calculated.

(2) Shearing cohesion of hexagonal boron nitride powder

The measurement was carried out with use of a powder bed shear stress analyzer NS-S500 (available from Nano Seeds Corporation) according to JISZ8835: 2016. Specifically, after a shearing cell was filled with hexagonal boron nitride powder and an upper surface of the powder bed was flattened, a side sliding was started with a target pressing load of 160 **N,** and a vertical stress and a shearing stress were measured over time, so that a powder yield locus was measured. Thereafter, the shearing cohesion was calculated from an intersection between the powder yield locus and the shearing stress axis.

(3) Elongation and content proportion of structure of hexagonal boron nitride powder

With use of a sieve having a mesh size of 25 $\mu$m, 15 g of hexagonal boron nitride powder was sieved. The hexagonal boron nitride powder that had not passed therethrough (powder residue on the sieve having a mesh size of 25 $\mu$m) was collected, and then the weight thereof was measured. Part of the powder residue on the sieve having a mesh size of 25 $\mu$m was observed with use of a scanning electron microscope (SEM). From an SEM image obtained, the length of the longest line (long axis) of the lines connecting the ends and the ends was measured as a long-axis diameter a, and the length of the longest line (short axis) of the straight lines that are perpendicular to the long axis was measured as a short-axis diameter b, in each structure. An elongation of each structure was then calculated. Thereafter, areas of the structures having elongations of not less than 0.80 and the areas of the other particles were calculated, and an area proportion of the structures having elongations of not less than 0.80 to the area of all the particles (sum of the areas of the structures having elongations of not less than 0.80 and the areas of the other particles) was calculated. This was regarded as the proportion of structures made of aggregates of primary hexagonal boron nitride particles having elongations of not less than 0.80.

(4) Aspect ratio of primary particle of hexagonal boron nitride powder

**In** 100 parts by mass of an epoxy resin (available from Henkel Ltd.; EA E-30CL), 10 parts by mass of the hexagonal boron nitride powder was dispersed. After a resin composition thus obtained was defoamed under reduced pressure, the resin composition was introduced into a formwork measuring 10 mm square 1 mm in thickness and was then cured at a temperature of 70°C.

Subsequently, the cured resin composition was drawn out of the formwork and was then polished so that both sides thereof were parallel. Thereafter, the center of one surface of the surfaces perpendicular to a thickness direction of the resin composition was subjected to a cross-sectional milling process, and an image of the surface subjected to the process was taken by an SEM under a condition of a magnification of 2500 times. From the image thus obtained, 100 primary boron nitride particles were randomly selected, and the long sides (major axes) and the short sides (thicknesses) of the particles were measured. The average of the long sides and the average of the short sides were respectively regarded as an average major axis ($\mu$m) and an average thickness ($\mu$m), and a value obtained by dividing the average major axis by the average thickness was regarded as an aspect ratio (average major axis/average thickness).

(5) Particle diameters (D50) and (D90) of hexagonal boron nitride powder

Measurement was carried out by a laser diffraction method with use of a laser diffraction/scattering type particle diameter measurement device MT3000 (available from MicrotracBEL Corp.). Specifically, 0.1 g of hexagonal boron nitride powder was introduced into a mixing vessel attached to the device filled with 50 cc of ethanol and was subjected to ultrasonic dispersion for 20 seconds at an output of 40 W to obtain a sample that was in turn used for measurement to calculate the volume-based particle diameters (D50) and (D90).

(6) Specific surface area of coarse hexagonal boron nitride powder

A gas adsorption test using nitrogen gas as an adsorption species was carried out with use of FlowSorb **III** 2310 (available from Micromeritics Instrument Corporation), and a nitrogen adsorption isothermal line was measured. Specifically, a continuous-flow method was carried out under a condition of a gas flow rate of 15 cm$^3$/min, on the coarse hexagonal boron nitride powder that had been subjected to vacuum drying deaeration at 200°C for 10 minutes as a pretreatment, so that an adsorption desorption isothermal line of the nitrogen gas was measured, and the specific surface area was calculated by the BET method.

(7) Eluted boron content of coarse hexagonal boron nitride powder and hexagonal boron nitride powder

Into 50-mL IBOY, 50 g of sulfuric acid aqueous solution having a concentration of 0.5 mol/L; and 2 g of coarse hexagonal boron nitride powder or hexagonal boron nitride powder were introduced and then were subjected to vibration stirring for one minute while adjusting the temperature of the solution to 25°C. Thereafter, the solution was left to stand for 120 minutes, and then a boron content in the solution thus obtained was analyzed with use of an ICP optical emission spectrometer (iCAP6500 available from THERMO FISHER Inc.). A measurement result thus obtained was divided by a mass of the coarse hexagonal boron nitride powder or hexagonal boron nitride powder used for the test, to calculate an amount (ppm) of eluted boron per unit mass.

Example 1

With use of a mixing stirrer, 120 g of boron oxide (B2O3) and 100 g of melamine (element ratio (B/N) of boron/nitrogen was 0.72) were mixed, and a capped carbon container was filled with the mixture, and the mixture was heated with use of a graphite Tammann furnace under a nitrogen gas atmosphere at 1000°C for 4 hours (reaction process). Thereafter, the nitride powder was obtained by increasing the temperature to reach 1750°C and then maintaining the temperature for 4 hours (firing process). Subsequently, the nitride powder thus obtained was disintegrated with use of a stone mill type pulverizer, and then washed with use of pure water, followed by being dried at 200°C. Thereafter, classification was carried out with use of a sieve having a mesh size of 45 $\mu$m, and the coarse hexagonal boron nitride powder having an eluted boron content of 4% was obtained.

The capped carbon container having a surface coated with boron nitride was filled with, as raw material powder, the coarse hexagonal boron nitride powder thus obtained so that a bulk density thereof became 0.19 g/cm$^3$, and then the coarse hexagonal boron nitride powder was heated with use of a graphite Tammann furnace under a nitrogen gas atmosphere at 1950°C for 6 hours. Thereafter, classification was carried out with use of a sieve having a mesh size of 45 μm, and hexagonal boron nitride powder was obtained. The production conditions and evaluation results of the hexagonal boron nitride powder thus obtained are shown in Table 1.

Examples 2 to 5 and Comparative Examples 2 to 4

Except that the production conditions were changed as shown in Table 1, hexagonal boron nitride powder was obtained in the same manner as Example 1. The eluted boron content in the coarse hexagonal boron nitride powder was adjusted by the washing condition. The production conditions and evaluation results of the hexagonal boron nitride powder thus obtained are shown in Table 1.

Comparative Example 1

The physical properties of the coarse hexagonal boron nitride powder obtained in Example 1 were evaluated. The production conditions and evaluation results of the hexagonal boron nitride powder thus obtained are shown in Table 1.

Loose foundations having the following compositions were produced with use of the hexagonal boron nitride powders obtained in Examples 1 to 5 and Comparative Examples 1 to 4 above.

| | |
|---|---|
| Hexagonal boron nitride powder | 20.0% by mass |
| Mica | 32.0% by mass |
| Synthetic phlogopite | 12.0% by mass |
| (vinyl dimethicone/methicone silsesquioxane)crosspolymer | 5.0% by mass |
| (diphenyl dimethicone/vinyl diphenyl dimethicone/silsesquioxane) crosspolymer | 5.0% by mass |
| Nylon 12 | 3.0% by mass |
| Silica | 3.0% by mass |
| Talc | 3.0% by mass |
| Zinc oxide | 3.0% by mass |
| Polymethyl methacrylate polymer | 3.0% by mass |
| Ethylhexyl methoxycinnamate | 2.0% by mass |
| Perfluorooctyltriethoxysilane | 1.0% by mass |
| Silicone-treated colcothar (red iron oxide) | 1.0% by mass |
| Silicone-treated yellow iron oxide | 0.6% by mass |
| Silicone-treated black iron oxide | 0.4% by mass |
| Silicone-treated titanium oxide | 6.0% by mass |

Sensory evaluation was carried out by 20 expert panelists as to airy feel and moist feel of a loose foundation obtained in accordance with the above formulation. The evaluation was regarded as "bad" in a case where less than 30% of the panelists felt that it was favorable, was regarded as "normal" in a case where not less than 30% and less than 60% of the panelists felt that it was favorable, was regarded as "good" in a case where not less than 60% and less than 80% of the panelists felt that it was favorable, and was regarded as "very good" in a case where not less than 80% of the panelists felt that it was favorable. The evaluation results are shown in Table 1.

Table 1

| | | | Unit | Example 1 | Example 2 | Example 3 |
|---|---|---|---|---|---|---|
| Production condition for coarse hexagonal boron nitride powder | Raw material mixture | $B_2O_3$ | g | 120 | 120 | 120 |
| | | Melamine | g | 100 | 100 | 100 |
| | | B/N | - | 0.72 | 0.72 | 0.72 |
| | Firing process condition | Maximum temperature | °C | 1750 | 1750 | 1750 |
| | | Retention time | hour | 4 | 4 | 4 |
| Production condition for hexagonal boron nitride powder | Physical property of coarse hexagonal boron nitride powder | Specific surface area | m²/g | 22 | 22 | 22 |
| | | Eluted boron content | % by mass | 4 | 4 | 4 |
| | Heating process condition | Bulk density of raw material powder | g/cm³ | 0.19 | 0.19 | 0.19 |
| | | Heating temperature | °C | 1950 | 1950 | 1750 |
| | | Retention time for heating | hour | 6 | 4 | 6 |
| Physical property of hexagonal boron nitride powder | | Tap bulk density | g/cm³ | 0.02 | 0.04 | 0.05 |
| | | Shearing cohesion | kPa | 4.7 | 4.3 | 3.5 |
| | | Proportion of structures made of aggregates of primary hexagonal boron nitride particles having elongation of not less than 0.80 | % | 13 | 16 | 13 |
| | | Proportion of powder residue on sieve of 25 μm | % by mass | 55 | 51 | 42 |
| | | Particle diameter D50 | μm | 23.2 | 21.2 | 19.2 |
| | | Particle diameter D90 | μm | 40.0 | 39.0 | 37.0 |
| | | Aspect ratio of primary particles | - | 17 | 17 | 15 |
| | | Eluted boron content | ppm | 15 | 18 | 19 |
| Evaluation result for cosmetic (loose foundation) | | Airy feel | - | very good | very good | very good |
| | | Moist feel | - | very good | very good | good |

(Continued)

| | | | Unit | Example 4 | Example 5 | Comparative Example 1 |
|---|---|---|---|---|---|---|
| Production condition for coarse hexagonal boron nitride powder | Raw material mixture | $B_2O_3$ | g | 120 | 200 | 120 |
| | | Melamine | g | 100 | 100 | 100 |
| | | B/N | - | 0.72 | 1.21 | 0.72 |
| | Firing process condition | Maximum temperature | °C | 1750 | 1900 | 1750 |
| | | Retention time | hour | 4 | 6 | 4 |

(continued)

| | | | Unit | Example 4 | Example 5 | Comparative Example 1 |
|---|---|---|---|---|---|---|
| Production condition for hexagonal boron nitride powder | Physical property of coarse hexagonal boron nitride powder | Specific surface area | m²/g | 22 | 15 | 22 |
| | | Eluted boron content | % by mass | 10 | 10 | 4 |
| | Heating process condition | Bulk density of raw material powder | g/cm³ | 0.19 | 0.22 | - |
| | | Heating temperature | °C | 1950 | 2000 | - |
| | | Retention time for heating | hour | 6 | 6 | - |
| Physical property of hexagonal boron nitride powder | | Tap bulk density | g/cm³ | 0.08 | 0.07 | 0.41 |
| | | Shearing cohesion | kPa | 3.8 | 3.5 | 2.5 |
| | | Proportion of structures made of aggregates of primary hexagonal boron nitride particles having elongation of not less than 0.80 | % | 18 | 14 | - |
| | | Proportion of powder residue on sieve of 25 μm | % by mass | 62 | 64 | 0 |
| | | Particle diameter D50 | μm | 29.4 | 35.0 | 3.5 |
| | | Particle diameter D90 | μm | 51.0 | 62.0 | 6.3 |
| | | Aspect ratio of primary particles | - | 18 | 13 | 10 |
| | | Eluted boron content | ppm | 19 | 9 | $4 \times 10^4$ |
| Evaluation result for cosmetic (loose foundation) | | Airy feel | - | good | good | bad |
| | | Moist feel | - | very good | good | bad |

(Continued)

| | | | Unit | Comparative Example 2 | Comparative Example 3 | Comparative Example 4 |
|---|---|---|---|---|---|---|
| Production condition for coarse hexagonal boron nitride powder | Raw material mixture | $B_2O_3$ | g | 120 | 120 | 120 |
| | | Melamine | g | 100 | 100 | 100 |
| | | B/N | - | 0.72 | 0.72 | 0.72 |
| | Firing process condition | Maximum temperature | °C | 1750 | 1750 | 1750 |
| | | Retention time | hour | 4 | 4 | 4 |

(continued)

|  | | | Unit | Comparative Example 2 | Comparative Example 3 | Comparative Example 4 |
|---|---|---|---|---|---|---|
| Production condition for hexagonal boron nitride powder | Physical property of coarse hexagonal boron nitride powder | Specific surface area | $m^2/g$ | 22 | 22 | 22 |
| | | Eluted boron content | % by mass | 0 | 4 | 4 |
| | Heating process condition | Bulk density of raw material powder | $g/cm^3$ | 0.19 | 0.12 | 0.25 |
| | | Heating temperature | °C | 1950 | 1950 | 1950 |
| | | Retention time for heating | hour | 6 | 6 | 6 |
| Physical property of hexagonal boron nitride powder | | Tap bulk density | $g/cm^3$ | 0.32 | 0.04 | 0.15 |
| | | Shearing cohesion | kPa | 2.9 | 3.0 | 3.5 |
| | | Proportion of structures made of aggregates of primary hexagonal boron nitride particles having elongation of not less than 0.80 | % | 6 | 9 | 12 |
| | | Proportion of powder residue on sieve of 25 $\mu$m | % by mass | 8 | 50 | 56 |
| | | Particle diameter D50 | $\mu$m | 9.3 | 20.2 | 23.8 |
| | | Particle diameter D90 | $\mu$m | 15.9 | 38.0 | 40.0 |
| | | Aspect ratio of primary particles | - | 15 | 17 | 18 |
| | | Eluted boron content | ppm | 16 | 16 | 16 |
| Evaluation result for cosmetic (loose foundation) | | Airy feel | - | bad | good | bad |
| | | Moist feel | - | bad | bad | good |

In the evaluation results, all the hexagonal boron nitride powders obtained in Examples 1 to 5 in which the heating container was filled with raw material powder containing coarse hexagonal boron nitride powder having an eluted boron content of 1% to 15% so that the bulk density became 0.16 $g/cm^3$ to 0.23 $g/cm^3$, and the raw material powder was heated at 1750°C to 2100°C for not less than 4 hours under a nitrogen atmosphere, so that the hexagonal boron nitride powder was obtained had tap bulk densities of not more than 0.08 $g/cm^3$ and powder shearing cohesions of not less than 3.5kPa. The loose foundations containing these hexagonal boron nitride powders showed favorable results as to both the airy feel and the moist feel, in the sensory evaluation.

In contrast, in Comparative Example 1 in which the coarse hexagonal boron nitride powder was evaluated without the heating treatment and in Comparative Example 2 in which the hexagonal boron nitride powder was produced by heating the coarse hexagonal boron nitride powder having an eluted boron content of less than 1%, the tap bulk densities exceeded 0.08 $g/cm^3$, shearing cohesions were less than 3.5 kPa, and neither the airy feel nor the moist feel was provided.

The hexagonal boron nitride powder obtained in Comparative Example 3 in which for the heating treatment on the coarse hexagonal boron nitride powder, the heating container was filled with the coarse hexagonal boron nitride powder so that the bulk density became less than 0.16 $g/cm^3$ to produce the hexagonal boron nitride powder had a shearing cohesion of less than 3.5 kPa and did not provide the moist feel. The hexagonal boron nitride powder obtained in Comparative Example 4 in which for the heating treatment on the coarse hexagonal boron nitride powder, the heating container was filled with the coarse hexagonal boron nitride powder so that the bulk density exceeded 0.23 $g/cm^3$ to produce the

hexagonal boron nitride powder had a tap bulk density exceeding 0.08 g/cm$^3$ and could not provide a favorable airy feel.

**Claims**

1. Hexagonal boron nitride powder having a tap bulk density of not more than 0.08 g/cm3 and a shearing cohesion of not less than 3.5 kPa, the shearing cohesion being calculated from a powder yield locus obtained through a powder bed shear stress analyzing test.

2. The hexagonal boron nitride powder according to claim 1, wherein in a powder residue on a sieve having a mesh size of 25 $\mu$m, a proportion of structures made of aggregates of primary hexagonal boron nitride particles having an elongation of not less than 0.80 is not less than 13%.

3. The hexagonal boron nitride powder according to claim 1, having a particle diameter D50 of 15 $\mu$m to 30 $\mu$m and an average aspect ratio of 15 to 20.

4. The hexagonal boron nitride powder according to claim 1, having a particle diameter D90 of 37 $\mu$m to 52 $\mu$m.

5. The hexagonal boron nitride powder according to any one of claims 1 to 4, used for a cosmetic application.

6. A cosmetic comprising the hexagonal boron nitride powder according to claim 5.

7. A loose foundation comprising the hexagonal boron nitride powder according to claim 5.

8. A method for producing hexagonal boron nitride powder, the method comprising a heating step of filling, with raw material powder containing coarse hexagonal boron nitride powder, a heating container so that a bulk density of the raw material powder becomes 0.16 g/cm$^3$ to 0.23 g/cm$^3$ and heating the raw material powder at a maximum temperature of 1700 °C to 2100 °C for not less than 3 hours under a nitrogen atmosphere, the coarse hexagonal boron nitride powder having a specific surface area of 12 m$^2$/g to 30 m$^2$/g and an eluted boron content of 1 % to 15 %.

FIG. 1

3.00kV x500 SE   100um

FIG. 2

3.00kV x500 SE   100um

## INTERNATIONAL SEARCH REPORT

| International application No. |
| --- |
| **PCT/JP2024/007518** |

| A. | CLASSIFICATION OF SUBJECT MATTER |
| --- | --- |

*C01B 21/064*(2006.01)i; *A61K 8/19*(2006.01)i; *A61Q 1/12*(2006.01)i
FI:  C01B21/064 M; C01B21/064 H; A61K8/19; A61Q1/12

According to International Patent Classification (IPC) or to both national classification and IPC

| B. | FIELDS SEARCHED |
| --- | --- |

Minimum documentation searched (classification system followed by classification symbols)

C01B21/064; A61K8/19; A61Q1/12; C04B35/5833; C08K3/38; C08L101/00; C08K9/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | WO 2022/224674 A1 (DENKA CO., LTD.) 27 October 2022 (2022-10-27)<br>entire text | 1-8 |
| A | WO 2022/085517 A1 (ADEKA CORPORATION) 28 April 2022 (2022-04-28)<br>entire text | 1-8 |
| A | WO 2021/039586 A1 (TOKUYAMA CORPORATION) 04 March 2021 (2021-03-04)<br>entire text | 1-8 |
| A | CN 103964403 A (NANJING UNIVERSITY OF AERONAUTICS AND ASTRONAUTICS)<br>06 August 2014 (2014-08-06)<br>entire text | 1-8 |

☐ Further documents are listed in the continuation of Box C.   ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **22 April 2024** | **07 May 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

EP 4 691 973 A1

**INTERNATIONAL SEARCH REPORT**
Information on patent family members

International application No.

**PCT/JP2024/007518**

| Patent document cited in search report | | | Publication date (day/month/year) | Patent family member(s) | | | Publication date (day/month/year) |
|---|---|---|---|---|---|---|---|
| WO | 2022/224674 | A1 | 27 October 2022 | CN | 117203155 | A | |
| | | | | KR | 10-2023-0169256 | A | |
| | | | | TW | 202308583 | A | |
| WO | 2022/085517 | A1 | 28 April 2022 | TW | 202233519 | A | |
| WO | 2021/039586 | A1 | 04 March 2021 | US | 2022/0281744 | A1 | |
| | | | | entire text | | | |
| | | | | EP | 4023207 | A1 | |
| | | | | CN | 114302858 | A | |
| | | | | KR | 10-2022-0054789 | A | |
| | | | | TW | 202114937 | A | |
| CN | 103964403 | A | 06 August 2014 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018108970 A

- WO 2019172440 A

**Non-patent literature cited in the description**

- *Bulletin of the Chemical Society of Japan*, 2020, vol. 93, 399-405